# EUROPEAN PATENT SPECIFICATION

(11) **EP 3 432 737 B1**
(45) Date of publication and mention of the grant of the patent: **22.01.2020**
(21) Application number: 17712969.9
(22) Date of filing: 22.03.2017
(51) Int. Cl.: A24F 47/00, H05B 3/42

(54) **ELECTRONIC VAPING DEVICE**
ELEKTRONISCHE VAPENVORRICHTUNG
DISPOSITIF ÉLECTRONIQUE DE VAPOTAGE

(30) Priority: 22.03.2016 US 201615076799
(43) Date of publication of application: 30.01.2019
(73) Proprietor: Philip Morris Products S.A., 2000 Neuchâtel (CH)
(72) Inventor: TUCKER, Christopher S., Midlothian, Virginia 23114 (US); SMITH, Barry S., Hopewell, Virginia 23860 (US); DENDY, Charles, Ruther Glen, Virginia 22546 (US)
(74) Representative: Dowling, Ian
(86) International application number: PCT/EP2017/056795
(87) International publication number: WO 2017/162724

(56) References cited:
- EP-A1- 2 574 247
- EP-A1- 2 787 846
- US-A1- 2014 196 733
- US-A1- 2015 272 218

## Description

The present disclosure relates to a heater assembly for an electronic vaping device and a method of making the electronic vaping device.

An electronic vaping device includes a heater element which vaporizes a pre-vapor formulation to produce a "vapor." The heater element may include a resistive heater coil, with a wick extending therethrough.

EP 2 787 846 A1 describes an aerosol generating device comprising a reservoir of aerosol-generating liquid positioned between an outer housing and an internal passageway. A vaporiser is located in the internal passageway, the passageway being lined with a porous interface. The vaporiser has an internal heater with a first electrical connection lead connected to one end.

According to a first aspect of the present invention there is provided a cartridge according to claim 1.

In at least one example embodiment, the heater is a coil heater. The cartridge may also include a chimney cage cap. The chimney cage cap may include a generally disc-shaped base, a cylindrical, longitudinally extending first tube including a slot in a first end of the first tube, the slot configured to receive a first end portion of the coil heater, and a cylindrical, longitudinally extending second tube configured to contact a first end of the chimney cage. The cathode clip includes at least one arm, the arm configured to contact the housing. The at least one arm may extend away from the heater assembly and may contact the housing. In at least one example embodiment, the at least one arm may extend between the at least one layer of gauze and the housing, such that the at least one arm contacts the housing.

According to a second aspect of the present invention, there is provided an electronic vaping device according to claim 7.

In at least one example embodiment, the electronic vaping device also includes a battery section. The battery section includes a battery, a controller, and a sensor. The heater is a coil heater. The electronic vaping device may also include a chimney cage cap. The chimney cage cap may include a generally disc-shaped base, a cylindrical, longitudinally extending first tube including a slot in a first end of the first tube, the slot configured to receive a first end portion of the coil heater, and a cylindrical, longitudinally extending second tube configured to contact a first end of the chimney cage.

In at least one example embodiment, the cathode clip includes at least one arm. The arm is configured to contact the housing. The at least one arm may extend away from the heater assembly and contacts the housing. The at least one arm may extend towards the heater assembly and between the at least one layer of gauze and the housing, such that the at least one arm contacts the housing.

According to a third aspect of the present invention, there is provided a method according to claim 14.

The method may further include folding the at least one arm such that the at least one arm extends away from the wrapped heater assembly, folding the at least one arm such that the at least one arm extends towards the wrapped heater assembly, or both. The method may also include removing the first mandrel, removing the second mandrel, and inserting a second connector in a second end of the wrapped heater assembly. In at least one example embodiment, the method may include injecting a pre-vapor formulation onto the at least one layer of gauze material, inserting a mouth-end insert in a first end of the housing, or both. In at least one example embodiment, the method may include threading the first electrical lead through a chimney cage cap, positioning the cathode clip on the chimney cage cap, welding the first electrical lead to the cathode clip, and combinations thereof.

The accompanying drawings are merely provided for illustrative purposes and should not be interpreted to limit the scope of the claims. The accompanying drawings are not to be considered as drawn to scale unless explicitly noted. For purposes of clarity, various dimensions of the drawings may have been exaggerated.
FIG. 1 is a side view of an electronic vaping device according to at least one example embodiment.
FIG. 2 is a cross-sectional view along line II-II of the electronic vaping device of FIG. 1.
FIG. 3 is an exploded view of a heater assembly according to at least one example embodiment.
FIG. 4 is an exploded view of a cartridge according to at least one example embodiment.
FIG. 5 is a cross-sectional view of a cartridge according to at least one example embodiment.
FIGS. 6A-6I illustrate a method of assembling a cartridge including a heater assembly according to at least one example embodiment.
FIGS. 7A-7E illustrate a method of assembling a cartridge including a heater assembly according to at least one example embodiment.
FIG. 8 is an illustration of a connector for a cartridge according to at least one example embodiment.

Some detailed example embodiments are disclosed herein. However, specific structural and functional details disclosed herein are merely representative for purposes of describing example embodiments. Example embodiments may, however, be embodied in many alternate forms and should not be construed as limited to only the example embodiments set forth herein.

Accordingly, while example embodiments are capable of various modifications and alternative forms, example embodiments thereof are shown by way of example in the drawings and will herein be described in detail. It should be understood, however, that there is no intent to limit example embodiments to the particular forms disclosed, but to the contrary, example embodiments are to cover all modifications, equivalents, and alternatives falling within the scope of the claims. Like numbers refer to like elements throughout the description of the figures.

It should be understood that when an element or layer is referred to as being "on," "connected to," "coupled to," or "covering" another element or layer, it may be directly on, connected to, coupled to, or covering the other element or layer or intervening elements or layers may be present. In contrast, when an element is referred to as being "directly on," "directly connected to," or "directly coupled to" another element or layer, there are no intervening elements or layers present. Like numbers refer to like elements throughout the specification.

It should be understood that, although the terms first, second, third, and so forth may be used herein to describe various elements, components, regions, layers or sections, these elements, components, regions, layers, or sections should not be limited by these terms. These terms are only used to distinguish one element, component, region, layer, or section from another element, component, region, layer, or section. Therefore, a first element, component, region, layer, or section discussed below could be termed a second element, component, region, layer, or section without departing from the teachings of example embodiments.

Spatially relative terms (for example, "beneath," "below," "lower," "above," "upper," and the like) may be used herein for ease of description to describe one element or feature's relationship to another element or feature as illustrated in the figures. It should be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Therefore, the term "below" may encompass both an orientation of above and below. The device may be otherwise oriented (rotated 90 degrees or at other orientations) and the spatially relative descriptors used herein interpreted accordingly.

The terminology used herein is for the purpose of describing various example embodiments only and is not intended to be limiting of example embodiments. As used herein, the singular forms "a," "an," and "the" are intended to include the plural forms as well, unless the context clearly indicates otherwise. It will be further understood that the terms "includes," "including," "comprises," and "comprising," when used in this specification, specify the presence of stated features, integers, steps, operations, elements, or components, but do not preclude the presence or addition of one or more other features, integers, steps, operations, elements, components, or groups thereof.

Example embodiments are described herein with reference to cross-sectional illustrations that are schematic illustrations of idealized embodiments (and intermediate structures) of example embodiments. As such, variations from the shapes of the illustrations as a result, for example, of manufacturing techniques or tolerances, are to be expected. Therefore, example embodiments should not be construed as limited to the shapes of regions illustrated herein but are to include deviations in shapes that result, for example, from manufacturing.

Unless otherwise defined, all terms (including technical and scientific terms) used herein have the same meaning as commonly understood by one of ordinary skill in the art to which example embodiments belong. It will be further understood that terms, including those defined in commonly used dictionaries, should be interpreted as having a meaning that is consistent with their meaning in the context of the relevant art and will not be interpreted in an idealized or overly formal sense unless expressly so defined herein.

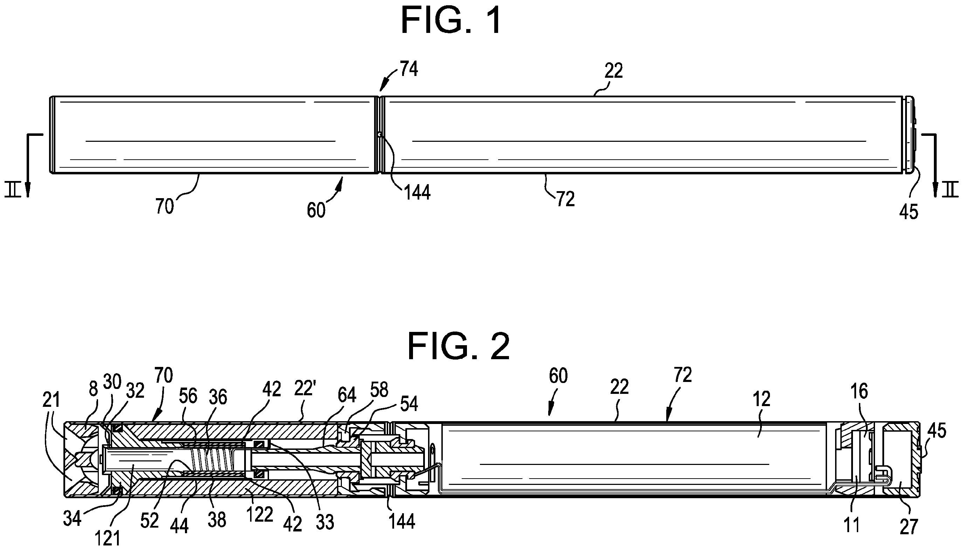

In at least one example embodiment, as shown in FIG. 1, the electronic vaping device 60 may include a replaceable cartridge (or first section) 70 and a reusable battery section (or second section) 72, which may be coupled together at a connector 74, which may be a threaded connector.

In at least one example embodiment, the connector 74 may be any type of connector, such as a snug-fit, detent, clamp, bayonet, clasp, or combinations thereof. The second section 72 may include a sensor 16 responsive to air drawn into the second section 72, a power supply 12, and a control circuit 11.

In at least one example embodiment, the first section 70 may include an outer housing 22' extending in a longitudinal direction. The second section 70 may include an outer housing 22 that also extends in the longitudinal direction.

In at least one example embodiment, the first section 70 includes a reservoir 122 that contains a pre-vapor formulation. The first section 70 may also include a heater 36 that is configured to vaporize the pre-vapor formulation.

The pre-vapor formulation may be a material or combination of materials that may be transformed into a vapor. For example, the pre-vapor formulation may be at least one of a liquid, solid, or gel formulation including, but not limited to, water, beads, solvents, active ingredients, ethanol, plant extracts, natural or artificial flavors, vapor formers, such as glycerin and propylene glycol, and combinations thereof.

Upon completing the connection between the first section 70 and the second section 72, the power supply 12 may be electrically connectable with the heater 36 of the first section 70 upon actuation of the sensor 16. Air is drawn primarily into the first section 70 through one or more air inlets 144, which may be located along the housing 22, 22' or at the connector 74.

The outer housing 22, 22' may have a generally cylindrical cross-section. In at least one example embodiment, the outer housing 22, 22' may have a generally triangular cross-section or square cross-section. In at least one example embodiment, the housing 22, 22' may have a greater circumference or dimensions at the tip end than at a mouth-end of the electronic vaping device 60 or vice versa. In at least one example embodiment, the housing 22, 22' is a single, unitary housing. In other example embodiments, the housing 22, 22' may include two or more pieces.

In at least one example embodiment, as shown in FIG. 2, the electronic vaping device 60 includes a mouth-end insert 8 configured to be inserted in an open, mouth-end of the housing 22'. The mouth-end insert 8 may include at least one outlet 21. In some example embodiments, the mouth-end insert 8 may include two or more outlets 21 that are angled outwardly with respect to the longitudinal axis of the electronic vaping device 60.

As shown in FIG. 2, in at least one example embodiment, the first section 22' houses a heater assembly 56. As shown in FIGS. 2, 3, and 4, the heater assembly 56 may include a chimney cage 38 extending in the longitudinal direction. The heater 36 may extend longitudinally within the chimney cage 38. A heater gauze 52, such as a gauze wrap, may be positioned or wrapped about an outer circumference of the heater 36. The chimney cage 38 is positioned about or fully or partially surrounds an outer circumference of the heater gauze 52. An inner gauze 42 and an outer gauze 44 may surround or be wrapped entirely or partially about an outer circumference of the chimney cage 38. The inner gauze 42 and the outer gauze 44 are configured to hold the pre-vapor formulation.

In at least one example embodiment, an air passage 121 extends through the heater assembly 56. The air passage 121 extends in the longitudinal direction and allows air to flow through the heater assembly 121 from the air inlets 144 to the outlets 21. The air passage 121 may extend through the heater 36, such that air flowing through the air passage mixes with vapor formed at the heater 36 and flows with the vapor to the outlets 21 of the mouth-end insert 8.

In at least one example embodiment, the heater assembly 56 is inserted in the housing 22'. An annular space between an inner surface of the housing 22' and the chimney cage 38 forms a reservoir 122 that contains the pre-vapor formulation. The inner gauze 42, the outer gauze 44, or both, are wrapped around the chimney cage 38 and are positioned in the reservoir 122. The inner gauze 42 and outer gauze 44 and the gauze wrapping 52 hold, wick, or hold and wick the pre-vapor formulation from the reservoir 122 to the heater 36. The inner gauze 42, the outer gauze 44, or both, may help reduce or minimize leakage of the pre-vapor formulation from the reservoir 122.

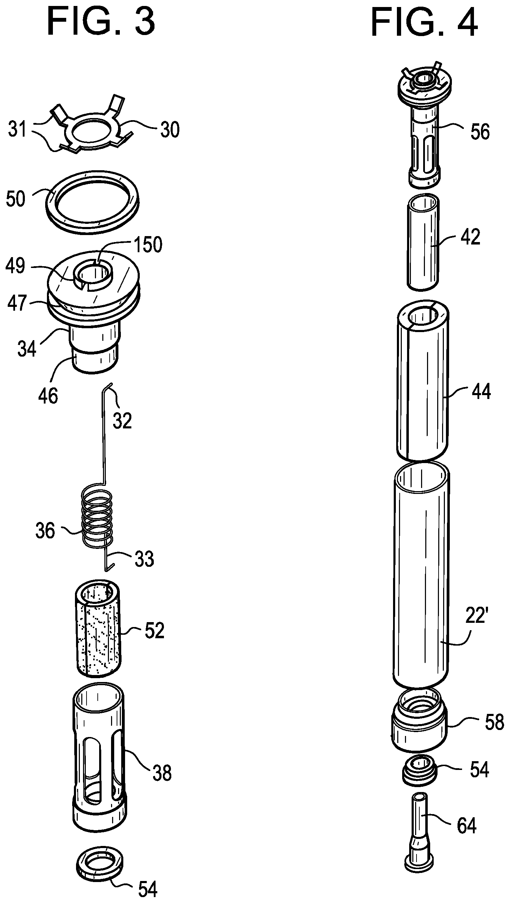

In at least one example embodiment, a cathode clip 30 is electrically connected to the heater 36. The cathode clip 30 may include at least one arm 31. As shown in Figures 3-4, the cathode clip 30 may include four arms 31. The at least one arm 31 is configured to contact the inner surface of the housing 22' when the cartridge is assembled. The at least one arm 31 may extend towards, away from, or towards and away from the heater assembly 56, and a length of the at least one arm 31 may vary and may be chosen to ensure contact with the inner surface of the outer housing 22'.

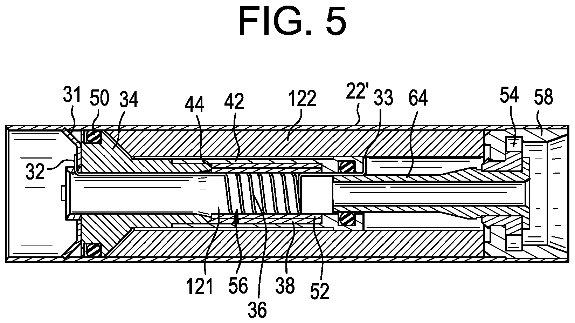

In at least one example embodiment, as shown in FIG. 5, the heater assembly 56 may also include a chimney cage cap 34 that is positioned at a first end of the heater assembly 56. The chimney cage cap 34 is configured to substantially prevent or reduce leakage of the pre-vapor formulation from the reservoir 122. In at least one example embodiment, a gasket 50 may surround a portion of the chimney cage cap 34. The gasket 50 reduces or minimizes leakage of the pre-vapor formulation from the reservoir 122.

In at least one example embodiment, the chimney cage cap 34 may include a base 47 that has a generally disc shape with a central hole extending there through. An outer edge of the base 47 may friction fit with an inner surface of the outer housing 22'. The chimney cage cap 34 may also include a first tube 49 and a second tube 46. Holes extending through the first tube 49 and the second tube 46 may align with the central hole in the base 47 such that air, vapor, or air and vapor may flow through the chimney cage cap 34. The first tube 49 and the second tube 46 are generally cylindrical in cross-section. The first tube 49 and the second tube 46 extend longitudinally within the housing 22'. The first tube 49 may include a slot 150 in a first end of the first tube 49. The slot 150 is configured to receive a first end portion or electrical lead 32 of the heater 36. Once the first end portion or electrical lead 32 of the heater 36 is inserted in the slot 150, the electrical lead 32 contacts the cathode clip 30 to electrically connect the heater 36 to the power supply 12. The electrical lead 32 may be spot welded to the cathode clip 30 if desired. The second tube 46 may be configured to contact a first end of the chimney cage 38.

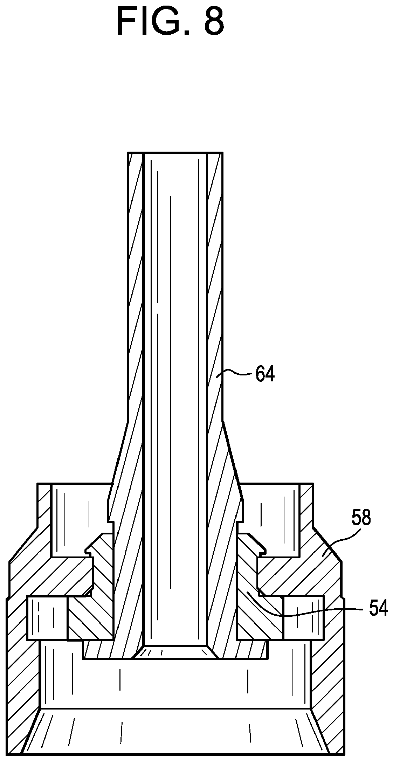

In at least one example embodiment, as shown in FIGS. 4 and 8, at a second end of the heater assembly 56, a nose portion of an anode 64 extends through an anode gasket 54 and a cathode connector 58. A second portion or electrical lead 33 extending from the heater 36 may be in contact with, welded to, or in contact with and welded to the anode so as to electrically connect the heater 36 and the power supply 12. The cathode connector 58 may include a threaded section for effecting the connection between the first section 70 and the battery section 72.

In at least one example embodiment, the anode gasket 54 may seal a second end of the reservoir 122 so as to reduce or prevent leakage of the pre-vapor formulation from the reservoir 122. In at least one example embodiment, an outer perimeter of the gasket 54 may provide a seal with an interior surface of the outer housing 22'. The gasket 54 may surround a portion of the cathode connector 58 and the anode 64.

In at least one example embodiment, as shown in FIG. 2, one or more air inlets 144 may be included in the outer housing 22, 22'. Alternatively, a single air inlet 144 may be included in the outer housing 22, 22'. The air inlets 144 may be placed adjacent to the connector 74 so as to prevent or reduce occlusion of the air inlets 144. In at least one example embodiment, the air inlets 144 may be provided in the connector 74.

As discussed above, in at least one example embodiment, the reservoir 122 may be contained in an outer annulus between the chimney cage 38 and an inner surface of the outer housing 22', and between the gasket 54 the chimney cage cap 34. Therefore, the reservoir 22 may at least partially surround the inner passage 121.

In at least one example embodiment, the reservoir 122 may be sized and configured to hold enough pre-vapor formulation such that the electronic vaping device 60 may be configured for vaping for at least about 200 seconds. Moreover, the electronic vaping device 60 may be configured to allow each puff to last a maximum of about 5 seconds. In at least one example embodiment, the electronic vaping device 60 may be configured to adjust maximum puff length.

As discussed above, the gauze wrapping 52, the inner gauze 42, the outer gauze 44, or combinations thereof, may contain, wick, or contain and wick the pre-vapor formulation. Therefore, the gauze wrapping 52, the inner gauze 42, the outer gauze 44, or combinations thereof, may comprise a storage medium, a material that wicks the pre-vapor formulation, or both. The storage medium may be a fibrous material including at least one of cotton, polyethylene, polyester, rayon and combinations thereof. The fibers may have a diameter ranging in size from about 6 microns to about 15 microns (for example, about 8 microns to about 12 microns or about 9 microns to about 11 microns). The storage medium may be a sintered, porous or foamed material. Also, the fibers may be sized to be irrespirable and may have a cross-section which has a Y-shape, cross shape, clover shape or any other suitable shape. A density of each of the gauze wrapping 52, the inner gauze 42, the outer gauze 44, or combinations thereof may be substantially the same or different. In at least one example embodiment, a thickness of each of the gauze wrapping 52, the inner gauze 42, the outer gauze 44, or combinations thereof, may be substantially the same or different. In at least one example embodiment, the gauze wrapping 52 may be a shaped tube of fibrous material that has a self-sustaining shape.

During vaping, pre-vapor formulation may be transferred from the reservoir 122, inner gauze 42 and outer gauze 44, or both, to the proximity of the heater 36 via capillary action of the gauze wrapping 52.

In at least one example embodiment, the gauze wrapping 52 may include any suitable material or combination of materials. The gauze wrapping 52 may have any suitable capillarity drawing action to accommodate pre-vapor formulations having different physical properties such as density, viscosity, surface tension and vapor pressure.

In at least one example embodiment, the heater 36 may include a wire coil. The wire may be a metal wire. The heater coil may extend fully or partially along the length of the inner passage 121 and air may flow through a central portion of the coil during vaping.

The heater coil may be formed of any suitable electrically resistive materials. Examples of suitable electrically resistive materials may include, but not limited to, titanium, zirconium, tantalum and metals from the platinum group. Examples of suitable metal alloys include, but not limited to, stainless steel, nickel, cobalt, chromium, aluminum-titanium-zirconium, hafnium, niobium, molybdenum, tantalum, tungsten, tin, gallium, manganese and iron-containing alloys, and super-alloys based on nickel, iron, cobalt, stainless steel. For example, the heater 36 may be formed of nickel aluminide, a material with a layer of alumina on the surface, iron aluminide and other composite materials, the electrically resistive material may optionally be embedded in, encapsulated or coated with an insulating material or vice-versa, depending on the kinetics of energy transfer and the external physicochemical properties required. The heater 36 may include at least one material selected from the group consisting of stainless steel, copper, copper alloys, nickel-chromium alloys, super alloys and combinations thereof. In an example embodiment, the heater 36 may be formed of nickel-chromium alloys or iron-chromium alloys. In another example embodiment, the heater 36 may be a ceramic heater having an electrically resistive layer on an outside surface thereof.

The heater 36 may heat pre-vapor formulation in the gauze wrapping 52 by thermal conduction. Alternatively, heat from the heater 36 may be conducted to the pre-vapor formulation by means of a heat conductive element or the heater 36 may transfer heat to the incoming ambient air that is drawn through the electronic vaping device 60 during vaping, which in turn heats the pre-vapor formulation by convection.

The power supply 12 may include a battery arranged in the electronic vaping device 60. The power supply 12 may be a Lithium-ion battery or one of its variants, for example a Lithium-ion polymer battery. Alternatively, the power supply 12 may be a nickel-metal hydride battery, a nickel cadmium battery, a lithium-manganese battery, a lithium-cobalt battery or a fuel cell. The electronic vaping device 60 may be usable by an adult vaper until the energy in the power supply 12 is depleted or in the case of lithium polymer battery, a minimum voltage cut-off level is achieved.

Further, the power supply 12 may be rechargeable and may include circuitry configured to allow the battery to be chargeable by an external charging device. To recharge the electronic vaping device 60, an USB charger or other suitable charger assembly may be used.

In at least one example embodiment, the sensor 16 may be configured to sense an air pressure drop and initiate application of voltage from the power supply 12 to the heater 36. The control circuit 11 may also include a heater activation light 27 configured to glow when the heater 36 is activated. The heater activation light 27 may include an LED and may be at a second end of the electronic vaping device 60. Moreover, the heater activation light 27 may be arranged to be visible to an adult vaper during vaping. In addition, the heater activation light 27 may be utilized for e-vaping system diagnostics or to indicate that recharging is in progress. The heater activation light 27 may also be configured such that the adult vaper may activate, deactivate, or activate and deactivate the heater activation light 27 for privacy. The heater activation light 27 may be on a tip end of the electronic vaping device 60 or on a side of the housing 22.

In at least one example embodiment, the control circuit 11 may supply power to the heater 36 responsive to the sensor 16. In at least one example embodiment, the control circuit 11 may include a maximum, time-period limiter. In another example embodiment, the control circuit 11 may include a manually operable switch for an adult vaper to initiate a puff. The time-period of the electric current supply to the heater 36 may be pre-set depending on the amount of pre-vapor formulation desired to be vaporized. In yet another example embodiment, the control circuit 11 may supply power to the heater 36 as long as the sensor 16 detects a pressure drop.

When activated, the heater 36 may heat a portion of the gauze wrapping 52 for less than about 10 seconds. Therefore, the power cycle (or maximum puff length) may range in period from about 2 seconds to about 10 seconds (for example, about 3 seconds to about 9 seconds, about 4 seconds to about 8 seconds or about 5 seconds to about 7 seconds).

In at least one example embodiment, the first section 70 may be replaceable. In other words, once the pre-vapor formulation is depleted, only the first section 70 may be replaced. In another example embodiment, the entire electronic vaping device 60 may be disposed once the reservoir 122 is depleted.

In at least one example embodiment, the power supply 12 may include a battery arranged in the electronic vaping device 60. The power supply 12 may be a Lithium-ion battery or one of its variants, for example a Lithium-ion polymer battery. Alternatively, the power supply 12 may be a nickel-metal hydride battery, a nickel cadmium battery, a lithium-manganese battery, a lithium-cobalt battery or a fuel cell. The electronic vaping device 60 may be usable by an adult vaper until the energy in the power supply 12 is depleted or in the case of lithium polymer battery, a minimum voltage cut-off level is achieved.

Further, the power supply 12 may be rechargeable and may include circuitry configured to allow the battery to be chargeable by an external charging device. To recharge the electronic vaping device 60, an USB charger or other suitable charger assembly may be used.

Further, the control circuit 11 may supply power to the heater 36 responsive to the sensor 16. In one example embodiment, the control circuit 11 may include a maximum, time-period limiter. In another example embodiment, the control circuit 11 may include a manually operable switch for an adult vaper to initiate a puff. The time-period of the electric current supply to the heater 36 may be pre-set depending on the amount of pre-vapor formulation desired to be vaporized. In yet another example embodiment, the control circuit 11 may supply power to the heater 36 as long as the sensor detects a pressure drop.

In an example embodiment, the electronic vaping device 60 may be about 80 millimetres to about 110 millimetres long and about 7 millimetres to about 8 millimetres in diameter. For example, in one example embodiment, the electronic vaping device 60 may be about 84 millimetres long and may have a diameter of about 7.8 millimetres.

In at least one example embodiment, not shown, the inner gauze 42 and the outer gauze 44 may be excluded from the electronic vaping device 60.

In at least one example embodiment, a method for automated manufacture of a cartridge of an electronic vaping device is provided. The method may result in a simplified device that reduces the complexity of manufacturing an electronic vaping device.

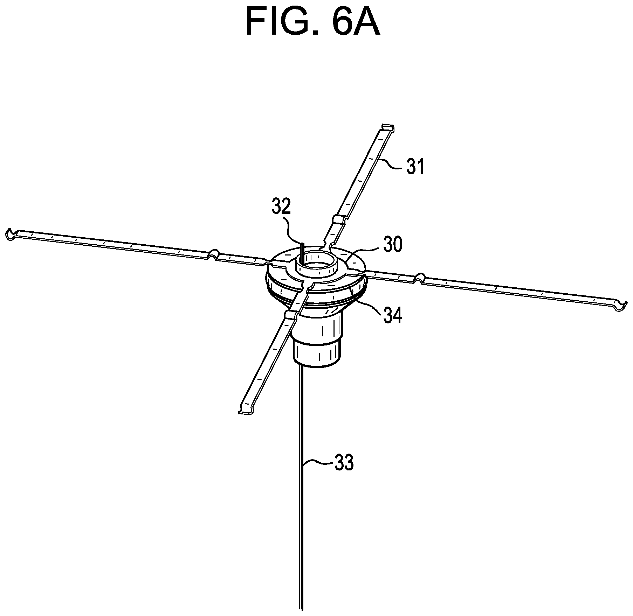

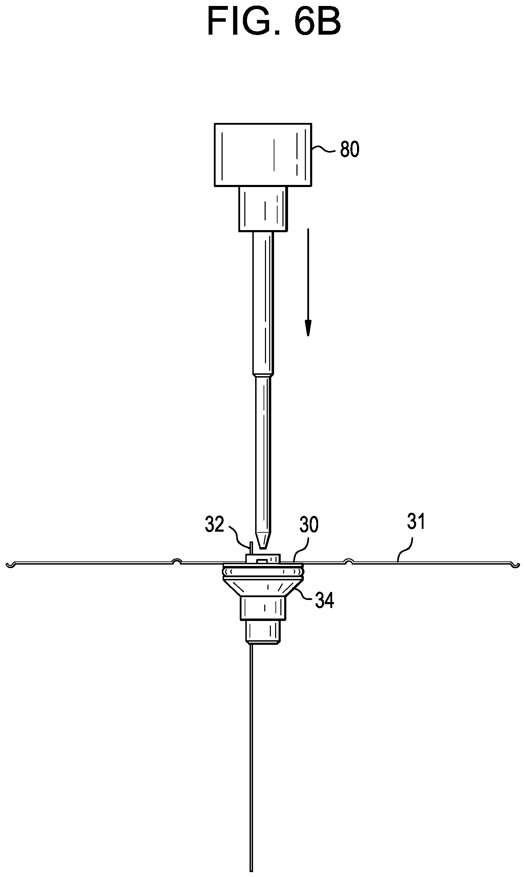

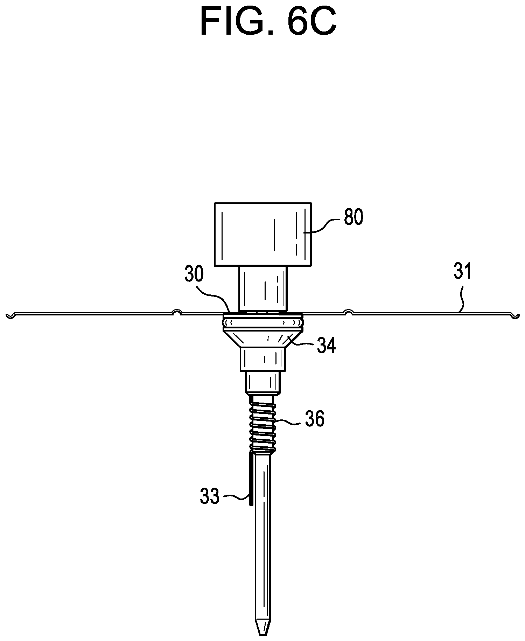

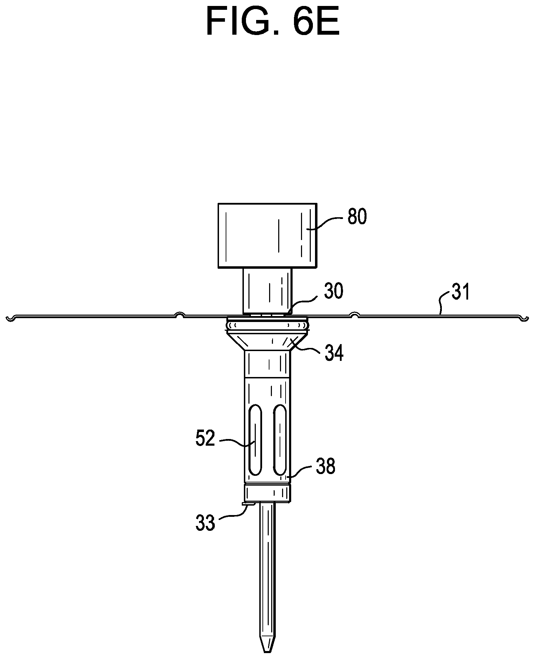

As shown in FIGS. 6A-6I, the method includes threading a first electrical lead 32 through a center of a chimney cage cap 34 and contacting the first electrical lead 32 with a cathode clip 30 including at least one arm 31. The method also includes inserting a first mandrel 80 through a center of the cathode clip 30 and through the center of the chimney cage cap 34. The method may include wrapping a heater coil around a stem of the first mandrel 80 as shown in FIG. 6C. The first mandrel 80 may include the stem portion that extends through the center of the heater 36 so as to maintain windings of the coil heater 36 in spaced apart relation, which may avoid or reduce hot spots during heating.

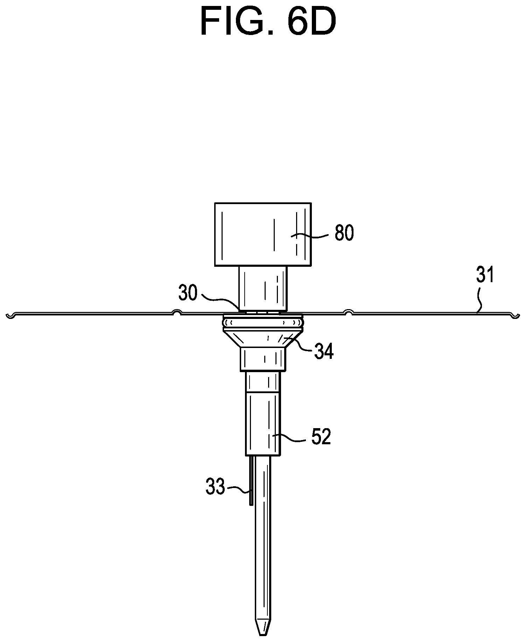

As shown in FIG. 6D, the method may further include wrapping a gauze material 52 around the heater 36 to form a wrapped heater. Once the heater 36 is wrapped, the method may include sliding a chimney cage 38 over the wrapped heater to form a heater assembly 56. The gauze material 52 may be wrapped tightly enough and secured around the heater 36 so that the chimney cage 38 slides over the wrapped heater.

In other example embodiments, a tongue or other implement may hold the gauze material 52 in place around the heater 36 while the chimney cage 38 is slid over the wrapper heater. The tongue may be withdrawn once the chimney cage 38 is in place. In another example embodiment, the chimney cage 38 could include a hinge mechanism (not shown) so that the chimney cage 38 is opened, positioned around the wrapped heater, and then secured there around.

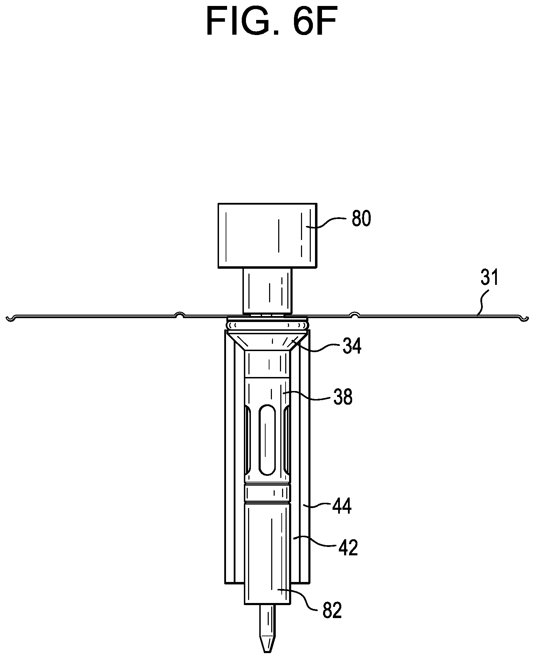

In at least one example embodiment, as shown in FIG. 6F, the method may also include inserting a second mandrel 82 into the heater 36 before or after positioning the chimney cage 38 around the heater 36.

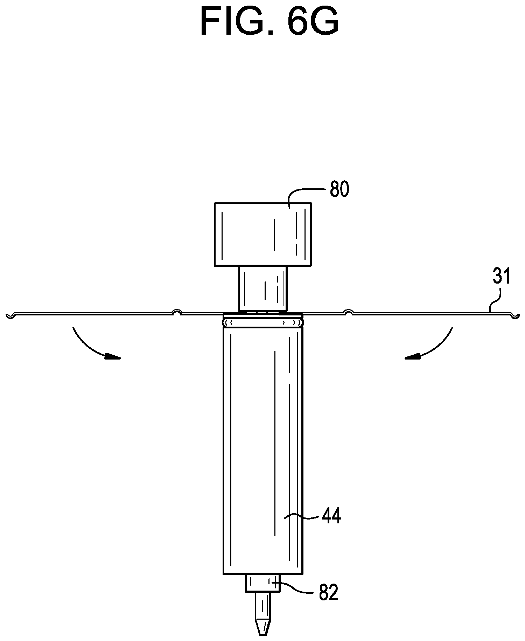

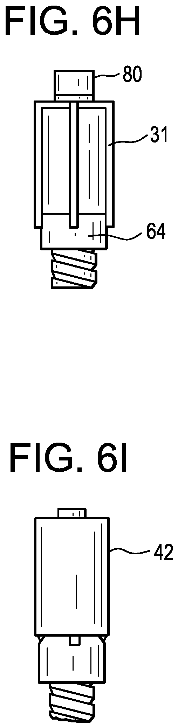

In at least one example embodiment, as shown in FIGS. 6G and 6H, the method may include folding the at least one arm 31 such that the at least one arm 31 extends towards the wrapped heater assembly, sliding a housing 22' over the wrapped heater assembly 56, or both. In another example embodiment, the at least one arm 31 is sized and configured to contact an inner surface of the housing 22' without being folded and therefore the method need not include the folding step.

In some example embodiments, as shown in FIG. 6I, the method may also include wrapping at least one layer of gauze material 42, 44 about the chimney cage 38 of the heater assembly 56 to form a wrapped heater assembly. The method may also include folding the at least one arm 31, such that the at least one arm 31 extends away from the wrapped heater assembly.

In at least one example embodiment, the method may include removing the first mandrel 80, removing the second mandrel 82, and inserting a second connector 58 in a second end of the wrapped heater assembly.

In at least one example embodiment, the method may also include injecting a pre-vapor formulation onto the at least one layer of gauze material 42, 44 after the heater assembly 56 is positioned within the outer housing 22' and the reservoir 122 is established between the chimney cage 38 and the outer housing 22'.

In at least one example embodiment, the method may include inserting a mouth-end insert 8 in a first end of the housing 22' after the mandrel 80 is removed.

In at least one example embodiment, the method may include threading the first electrical lead 32 through a slot 150 in a chimney cage cap 34, positioning the cathode clip 30 on the chimney cage cap 34, welding the first electrical lead 32 to the cathode clip 30, and combinations thereof. In at least one example embodiment, the electrical lead 32 need not be welded to the cathode clip 34, but maintains contact with the cathode clip 34 due to the positioning of the electrical lead 32 through the slot 150.

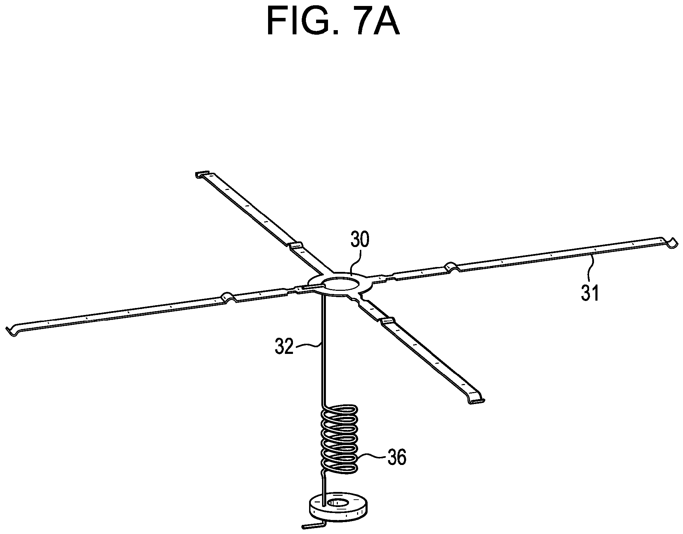

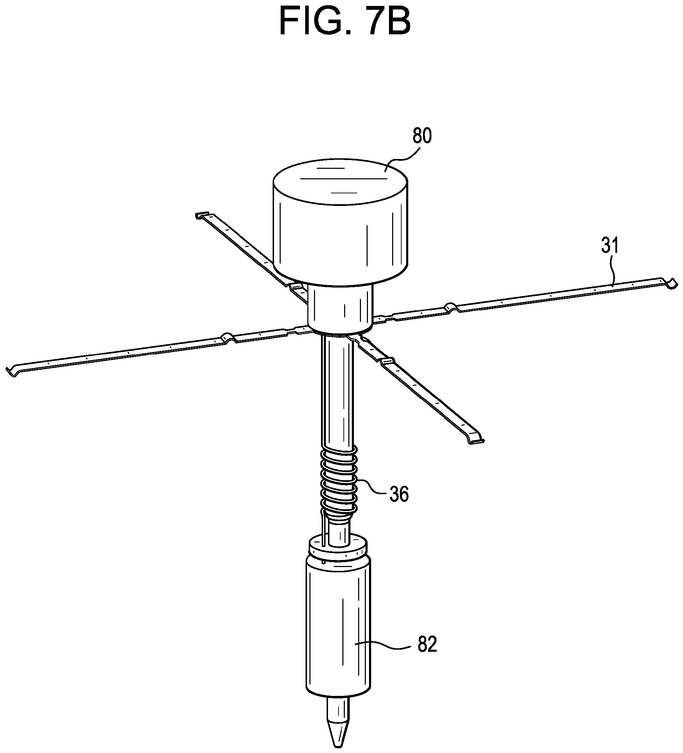

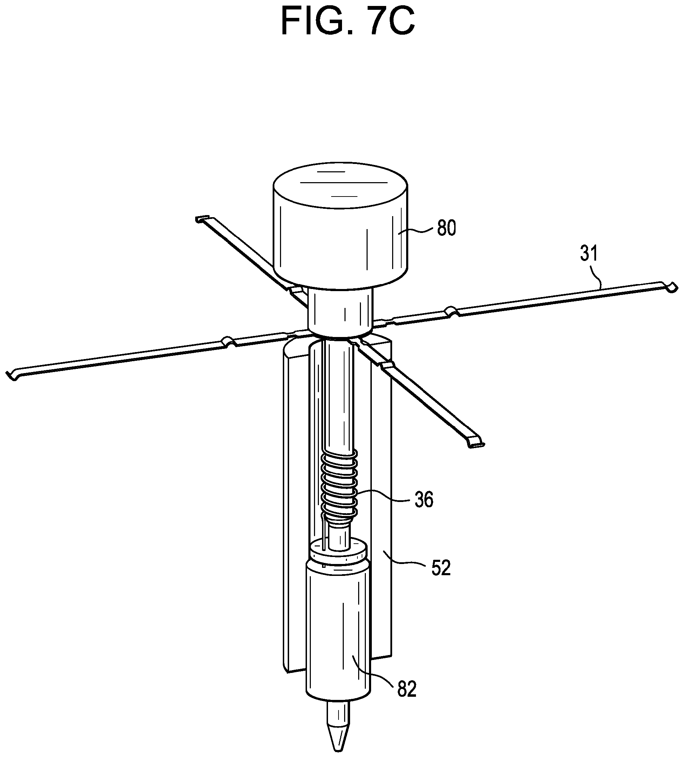

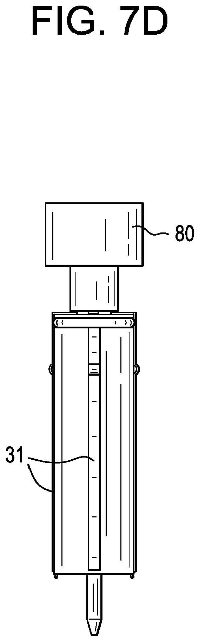

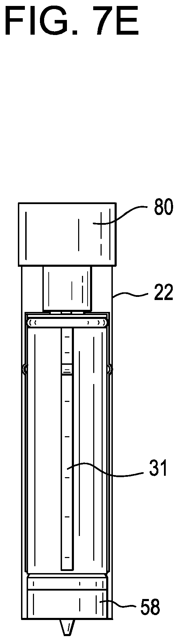

In at least one example embodiment, as shown in FIGS. 7A-7E, the method may be substantially the same as in FIGS. 6A-6I, but may include inserting the first mandrel 80 through a first end of the heater 36 instead of wrapping a heater coil around the first mandrel 80.

While a number of example embodiments have been disclosed herein, it should be understood that other variations may be possible. Such variations are not to be regarded as a departure from the scope of the present invention, and all such modifications as would be obvious to one skilled in the art are intended to be included within the scope of the following claims.

## Claims

1. A cartridge (70) for
an electronic vaping device (60), the cartridge (70) comprising:
a heater assembly (56) comprising,
a chimney cage (38) extending longitudinally;
a heater (36) extending longitudinally within the chimney cage (38);
a heater gauze (52) positioned about an outer circumference of the heater (36), the chimney cage (38) positioned about an outer circumference of the heater gauze (52); and
a cathode clip (30) electrically connected to a first end portion of the heater (36); and
a reservoir (122) configured to contain a pre-vapor formulation, the reservoir (122) comprising,
at least one layer of gauze (42, 44) circumscribing the chimney cage (38); and
a housing (22'), the reservoir (122) being positioned between the heater assembly (56) and an inner surface of the housing (22').

2. The cartridge (70) of claim 1, wherein the heater (36) is a coil heater.

3. The cartridge (70) of claim 1 or 2, further comprising:
a chimney cage cap (34), the chimney cage cap (34) including,
a generally disc-shaped base (47),
a cylindrical, longitudinally extending first tube (49) including a slot (150) in a first end of the first tube (49), wherein a first end portion of the heater (36) is received in the slot (150), and
a cylindrical, longitudinally extending second tube (46), wherein the second tube (46) contacts a first end of the chimney cage (38).

4. The cartridge (70) of claim 1, 2 or 3, wherein the cathode clip (30) includes at least one arm (31), wherein the at least one arm (31) contacts the housing (22').

5. The cartridge (70) of claim 4, wherein the at least one arm (31) extends away from the heater assembly (56) and contacts the housing (22').

6. The cartridge (70) of claim 4, wherein the at least one arm (31) extends between the at least one layer of gauze (42, 44) and the housing (22'), such that the at least one arm (31) contacts the housing (22').

7. An electronic vaping device (60), comprising:
a cartridge (70), the cartridge (70) including,
a heater assembly (56) including,
a chimney cage (38) extending longitudinally;
a heater (36) extending longitudinally within the chimney cage (38);
a heater gauze (52) positioned about an outer circumference of the heater (36), the chimney cage (38) positioned about an outer circumference of the heater gauze (52); and
a cathode clip (30) electrically connected to a first end portion of the heater (36); and
a reservoir (122) configured to contain a pre-vapor formulation, the reservoir (122) including,
at least one layer of gauze (42, 44) circumscribing the chimney cage (38);
and
a housing (22'), the reservoir (122) being positioned between the heater assembly (56) and an inner surface of the housing (22'); and
a battery section (72).

8. The electronic vaping device (60) of claim 7, the battery section (72) comprising:
a battery (12);
a controller (11); and
a sensor (16).

9. The electronic vaping device (60) of claim 7 or 8, wherein the heater (36) is a coil heater.

10. The electronic vaping device (60) of claim 7, 8 or 9, further comprising:
a chimney cage cap (34), the chimney cage cap (34) including,
a generally disc-shaped base (47),
a cylindrical, longitudinally extending first tube (49) including a slot (150) in a first end of the first tube (49), wherein a first end portion of the heater (36) is received in the slot (150), and
a cylindrical, longitudinally extending second tube (46), wherein the second tube (46) contacts a first end of the chimney cage (38).

11. The electronic vaping device (60) of any of claims 7 to 10, wherein the cathode clip (30) includes at least one arm (31), wherein the at least one arm (31) contacts the housing (22').

12. The electronic vaping device (60) of claim 11, wherein the at least one arm (31) extends away from the heater assembly (56) and contacts the housing (22').

13. The electronic vaping device (60) of claim 11, wherein the at least one arm (31) extends between the at least one layer of gauze (42, 44) and the housing (22'), such that the at least one arm (31) contacts the housing (22').

14. A method for automated manufacture of a cartridge (70) for an electronic vaping device (60), the method comprising:
contacting a cathode clip (30) including at least one arm (31) with a first electrical lead (32) extending from a heater (36);
inserting a first mandrel (80) in a first end of the heater (36);
inserting a second mandrel (82) in a second end of the heater (36);
wrapping a heater gauze (52) around the heater (36) to form a wrapped heater;
sliding a chimney cage (38) over the wrapped heater to form a heater assembly (56);
wrapping at least one layer of gauze material (42, 44) about the heater assembly (56) to form a wrapped heater assembly; and
sliding a housing (22') over the wrapped heater assembly.

15. The method of claim 14, further comprising:
folding the at least one arm (31), such that the at least one arm (31) extends away from the wrapped heater assembly.

16. The method of claim 14, further comprising:
folding the at least one arm (31), such that the at least one arm (31) extends towards the wrapped heater assembly.

17. The method of claim 14, 15 or 16, further comprising:
removing the first mandrel (80);
removing the second mandrel (82); and
inserting a connector (58) in an end of the wrapped heater assembly.

18. The method of claim 17, further comprising:
injecting a pre-vapor formulation onto the at least one layer of gauze material (42, 44).

19. The method of claim 18, further comprising:
inserting a mouth-end insert (8) in a first end of the housing (22').

20. The method of any of claims 14 to 19, further comprising:
threading the first electrical lead (32) through a chimney cage cap (34);
positioning the cathode clip (30) on the chimney cage cap (34); and
welding the first electrical lead (32) to the cathode clip (30).

## Patentansprüche

1. Patrone (70) für eine elektronische Dampfvorrichtung (60), wobei die Patrone (70) aufweist:
eine Heizvorrichtungsbaugruppe (56) aufweisend,
einen Kaminkäfig (38), der sich in Längsrichtung erstreckt;
eine Heizvorrichtung (36), die sich innerhalb des Kaminkäfigs (38) in Längsrichtung erstreckt;
eine Heizvorrichtungsgaze (52), die über einem äußeren Umfang der Heizvorrichtung (36) positioniert ist, wobei der Kaminkäfig (38) um einen äußeren Umfang der
Heizvorrichtungsgaze (52) herum positioniert ist; und
einen Kathodenclip (30), der mit einem ersten Endabschnitt der Heizvorrichtung (36) elektrisch verbunden ist; und
einen Vorratsbehälter (122), der ausgelegt ist, eine Vordampfformulierung zu enthalten, wobei der Vorratsbehälter (122) aufweist,
mindestens eine Gazeschicht (42, 44), die den Kaminkäfig (38) abgrenzt; und
ein Gehäuse (22'), wobei der Vorratsbehälter (122), zwischen der Heizvorrichtungsbaugruppe (56) und einer Innenfläche des Gehäuses (22') positioniert ist.

2. Patrone (70) nach Anspruch 1, wobei die Heizvorrichtung (36) eine Spulenheizvorrichtung ist.

3. Patrone (70) nach Anspruch 1 oder 2, weiter aufweisend:
eine Kaminkäfigkappe (34), wobei die Kaminkäfigkappe (34) einschließt
eine generell scheibenförmige Basis (47),
ein zylindrisches sich in Längsrichtung erstreckendes erstes Rohr (49) einschließlich eines Schlitzes (150) in einem ersten Ende des ersten Rohrs (49), wobei ein erster Endabschnitt der Heizvorrichtung (36) in dem Schlitz (150) aufgenommen ist, und
ein zylindrisches sich in Längsrichtung erstreckendes zweites Rohr (46), wobei das zweite Rohr (46) ein erstes Ende des Kaminkäfigs (38) kontaktiert.

4. Patrone (70) nach Anspruch 1, 2 oder 3, wobei der Kathodenclip (30) mindestens einen Arm (31) einschließt, wobei der mindestens eine Arm (31) das Gehäuse (22') kontaktiert.

5. Patrone (70) nach Anspruch 4, wobei der mindestens eine Arm (31) sich von der Heizvorrichtungsbaugruppe (56) weg erstreckt und das Gehäuse (22)' kontaktiert.

6. Patrone (70) nach Anspruch 4, wobei sich der mindestens eine Arm (31) zwischen der mindestens einen Gazeschicht (42, 44) und dem Gehäuse (22') derart erstreckt, dass der mindestens eine Arm (31) das Gehäuse (22') kontaktiert.

7. Elektronische Dampfvorrichtung (60), aufweisend:
eine Patrone (70), wobei die Patrone (70) einschließt,
eine Heizvorrichtungsbaugruppe (56) einschließlich
eines Kaminkäfigs (38), der sich in Längsrichtung erstreckt;
eine Heizvorrichtung (36), die sich innerhalb des Kaminkäfigs (38) in Längsrichtung erstreckt;
eine Heizvorrichtungsgaze (52), die über einem äußeren Umfang der Heizvorrichtung (36) positioniert ist, wobei der Kaminkäfig (38) um einen äußeren Umfang der Heizvorrichtungsgaze (52) herum positioniert ist; und
einen Kathodenclip (30), der mit einem ersten Endabschnitt der Heizvorrichtung (36) elektrisch verbunden ist; und
einen Vorratsbehälter (122), der ausgelegt ist, eine Vordampfformulierung zu enthalten, wobei der Vorratsbehälter (122) einschließt,
mindestens eine Gazeschicht (42, 44), die den Kaminkäfig (38) abgrenzt; und
ein Gehäuse (22'), wobei der Vorratsbehälter (122) zwischen der Heizvorrichtungsbaugruppe (56) und einer Innenfläche des Gehäuses (22') positioniert ist; und
einen Batterieteil (72).

8. Elektronische Dampfvorrichtung (60) nach Anspruch 7, wobei der Batterieteil (72) aufweist:
eine Batterie (12);
eine Steuerung (11); und
einen Sensor (16).

9. Elektronische Dampfvorrichtung (60) nach Anspruch 7 oder 8, wobei die Heizvorrichtung (36) eine Spulenheizvorrichtung ist.

10. Elektronische Dampfvorrichtung (60) nach Anspruch 7, 8 oder 9, weiter aufweisend:
eine Kaminkäfigkappe (34), wobei die Kaminkäfigkappe (34) einschließt
eine generell scheibenförmige Basis (47),
ein zylindrisches sich in Längsrichtung erstreckendes erstes Rohr (49) einschließlich eines Schlitzes (150) in einem ersten Ende des ersten Rohrs (49), wobei ein erster Endabschnitt der Heizvorrichtung (36) in dem Schlitz (150) aufgenommen ist, und
ein zylindrisches sich in Längsrichtung erstreckendes zweites Rohr (46), wobei das zweite Rohr (46) ein erstes Ende des Kaminkäfigs (38) kontaktiert.

11. Elektronische Dampfvorrichtung (60) nach einem der Ansprüche 7 bis 10, wobei der Kathodenclip (30) mindestens einen Arm (31) einschließt, wobei der mindestens ein Arm (31) das Gehäuse (22') kontaktiert.

12. Elektronische Dampfvorrichtung (60) nach Anspruch 11, wobei der mindestens eine Arm (31) sich von der Heizvorrichtungsbaugruppe (56) weg erstreckt und das Gehäuse (22') kontaktiert.

13. Elektronische Dampfvorrichtung (60) nach Anspruch 11, wobei sich der mindestens eine Arm (31) zwischen der mindestens einen Gazeschicht (42, 44) und dem Gehäuse (22') derart erstreckt, dass der mindestens eine Arm (31) das Gehäuse (22') kontaktiert.

14. Verfahren für automatisierte Herstellung einer Patrone (70) für eine elektronische Dampfvorrichtung (60), wobei das Verfahren aufweist:
Kontaktieren eines Kathodenclips (30) einschließlich mindestens eines Arms (31) mit einer ersten elektrischen Leitung (32), die sich von einer Heizvorrichtung (36) erstreckt;
Einsetzen eines ersten Dorns (80) in ein erstes Ende der Heizvorrichtung (36);
Einsetzen eines zweiten Dorns (82) in ein zweites Ende der Heizvorrichtung (36);
Hüllen einer Heizvorrichtungsgaze (52) um die Heizvorrichtung (36) herum, um eine umhüllte Heizvorrichtung zu bilden;
Schieben eines Kaminkäfigs (38) über die umhüllte Heizvorrichtung, um eine Heizvorrichtungsbaugruppe (56) zu bilden;
Hüllen von mindestens einer Gazematerialschicht (42, 44) um die Heizvorrichtungsbaugruppe (56) herum, um eine umhüllte Heizvorrichtungsbaugruppe zu bilden; und
Schieben eines Gehäuses (22') über die umhüllte Heizvorrichtungsbaugruppe.

15. Verfahren nach Anspruch 14, weiter aufweisend:
Falten des mindestens einen Arms (31), sodass sich der mindestens eine Arm (31) von der umhüllten Heizvorrichtungsbaugruppe weg erstreckt.

16. Verfahren nach Anspruch 14, weiter aufweisend:
Falten des mindestens einen Arms (31), sodass sich der mindestens eine Arm (31) in Richtung der umhüllten Heizvorrichtungsbaugruppe erstreckt.

17. Verfahren nach Anspruch 14, 15 oder 16, weiter aufweisend:
Entfernen des ersten Dorns (80);
Entfernen des zweiten Dorns (82); und
Einsetzen eines Verbinders (58) in ein Ende der umhüllten Heizvorrichtungsbaugruppe.

18. Verfahren nach Anspruch 17, weiter aufweisend:
Einspritzen einer Vordampfformulierung auf die mindestens eine Gazematerialschicht (42, 44).

19. Verfahren nach Anspruch 18, weiter aufweisend:
Einsetzen eines Mundendeeinsatzes (8) in ein erstes Gehäuseende (22').

20. Verfahren nach einem der Ansprüche 14 bis 19, weiter aufweisend:
Fädeln der ersten elektrischen Leitung (32) durch eine Kaminkäfigkappe (34);
Positionieren des Kathodenclips (30) an der Kaminkäfigkappe (34) ; und
Schweißen der ersten elektrischen Leitung (32) an den Kathodenclip (30).

## Revendications

1. Cartouche (70) pour un dispositif électronique de vapotage (60), la cartouche (70) comprenant :
un ensemble de chauffage (56) comprenant,
une cage de cheminée (38) s'étendant longitudinalement ;
un dispositif de chauffage (36) s'étendant longitudinalement dans la cage de cheminée (38) ;
une gaze du dispositif de chauffage (52) positionnée autour d'une circonférence extérieure du dispositif de chauffage (36), la cage de cheminée (38) positionnée autour d'une circonférence extérieure de la gaze du dispositif de chauffage (52) ; et
un clip de cathode (30) relié électriquement à une première partie d'extrémité du dispositif de chauffage (36) ; et
un réservoir (122) configuré pour contenir une formulation pré-vapeur, le réservoir (122) comprenant,
au moins une couche de gaze (42, 44) entourant la cage de cheminée (38) ; et
un logement (22'), le réservoir (122) étant positionné entre l'ensemble de chauffage (56) et une surface intérieure du logement (22').

2. Cartouche (70) selon la revendication 1, dans laquelle le dispositif de chauffage (36) est un dispositif de chauffage à bobine.

3. Cartouche (70) selon la revendication 1 ou 2, comprenant en outre :
un capuchon de la cage de cheminée (34), le capuchon de la cage de cheminée (34) incluant,
une base généralement en forme de disque (47),
un premier tube cylindrique, s'étendant longitudinalement (49), incluant une rainure (150) dans une première extrémité du premier tube (49), dans lequel une première partie d'extrémité du dispositif de chauffage (36) est reçue dans la rainure (150), et
un deuxième tube cylindrique, s'étendant longitudinalement (46), dans lequel le deuxième tube (46) est en contacte avec une première extrémité de la cage de cheminée (38).

4. Cartouche (70) selon la revendication 1, 2 ou 3, dans laquelle le clip de cathode (30) inclut au moins un bras (31), dans laquelle l'au moins un bras (31) est en contacte avec le logement (22').

5. Cartouche (70) selon la revendication 4, dans laquelle l'au moins un bras (31) s'étend à l'écart de l'ensemble de chauffage (56) et est en contacte avec le logement (22').

6. Cartouche (70) selon la revendication 4, dans laquelle l'au moins un bras (31) s'étend entre l'au moins une couche de gaze (42, 44) et le logement (22'), de sorte que l'au moins un bras (31) est en contacte avec le logement (22').

7. Dispositif de vapotage électronique (60), comprenant :
une cartouche (70), la cartouche (70), incluant,
un ensemble de chauffage (56), incluant,
une cage de cheminée (38) s'étendant longitudinalement ;
un dispositif de chauffage (36) s'étendant longitudinalement dans la cage de cheminée (38) ;
une gaze du dispositif de chauffage (52) positionnée autour d'une circonférence extérieure du dispositif de chauffage (36), la cage de cheminée (38) positionnée autour d'une circonférence extérieure de la gaze du dispositif de chauffage (52) ; et
un clip de cathode (30) relié électriquement à une première partie d'extrémité du dispositif de chauffage (36) ; et
un réservoir (122) configuré pour contenir une formulation pré-vapeur, le réservoir (122), incluant,
au moins une couche de gaze (42, 44) entourant la cage de cheminée (38) ; et
un logement (22'), le réservoir (122) étant positionné entre l'ensemble de chauffage (56) et une surface intérieure du logement (22') ; et
une section de batterie (72).

8. Dispositif de vapotage électronique (60) selon la revendication 7, la section de batterie (72) comprenant :
une batterie (12) ;
un contrôleur (11) ; et
un capteur (16).

9. Dispositif de vapotage électronique (60) selon la revendication 7 ou 8, dans lequel le dispositif de chauffage (36) est un dispositif de chauffage à bobine.

10. Dispositif de vapotage électronique (60) selon la revendication 7, 8 ou 9, comprenant en outre :
un capuchon de la cage de cheminée (34), le capuchon de la cage de cheminée (34) incluant,
une base généralement en forme de disque (47),
un premier tube cylindrique, s'étendant longitudinalement (49), incluant une rainure (150) dans une première extrémité du premier tube (49), dans lequel une première partie d'extrémité du dispositif de chauffage (36) est reçue dans la rainure (150), et
un deuxième tube cylindrique, s'étendant longitudinalement (46), dans lequel le deuxième tube (46) est en contacte avec une première extrémité de la cage de cheminée (38).

11. Dispositif de vapotage électronique (60) selon l'une quelconque des revendications 7 à 10, dans lequel le clip de cathode (30) inclut au moins un bras (31), dans lequel l'au moins un bras (31) est en contacte avec le logement (22').

12. Dispositif de vapotage électronique (60) selon la revendication 11, dans lequel l'au moins un bras (31) s'étend à l'écart de l'ensemble de chauffage (56) et est en contacte avec le logement (22').

13. Dispositif de vapotage électronique (60) selon la revendication 11, dans lequel l'au moins un bras (31) s'étend entre l'au moins une couche de gaze (42, 44) et le logement (22'), de sorte que l'au moins un bras (31) est en contacte avec le logement (22').

14. Procédé pour la fabrication automatisée d'une cartouche (70) pour un dispositif de vapotage électronique (60), le procédé comprenant :
le contact d'un clip de cathode (30) incluant au moins un bras (31) avec un premier fil électrique (32) s'étendant d'un dispositif de chauffage (36) ;
l'insertion d'un premier mandrin (80) dans une première extrémité du dispositif de chauffage (36) ;
l'insertion d'un deuxième mandrin (82) dans une deuxième extrémité du dispositif de chauffage (36) ;
l'emballage d'une gaze du dispositif de chauffage (52) autour du dispositif de chauffage (36) pour former un dispositif de chauffage enveloppé ;
le glissement d'une cage de cheminée (38) sur le dispositif de chauffage enveloppé pour former un ensemble de chauffage (56) ;
l'enveloppement d'au moins une couche de matière de gaze (42, 44) autour l'ensemble de chauffage (56) pour former un ensemble de chauffage enveloppé ; et
le glissement d'un logement (22') sur l'ensemble de chauffage enveloppé.

15. Procédé selon la revendication 14, comprenant en outre :
le pliage de l'au moins un bras (31), de sorte que l'au moins un bras (31) s'étend à l'écart de l'ensemble de chauffage enveloppé.

16. Procédé selon la revendication 14, comprenant en outre :
le pliage de l'au moins un bras (31), de sorte que l'au moins un bras (31) s'étend vers l'ensemble de chauffage enveloppé.

17. Procédé selon la revendication 14, 15 ou 16, comprenant en outre :
le retrait du premier mandrin (80) ;
le retrait du deuxième mandrin (82) ; et
l'insertion d'un connecteur (58) dans une extrémité de l'ensemble de chauffage enveloppé.

18. Procédé selon la revendication 17, comprenant en outre :
l'injection d'une formulation pré-vapeur sur l'au moins une couche de matériau de gaze (42, 44).

19. Procédé selon la revendication 18, comprenant en outre :
l'insertion d'un insert d'embout buccal (8) dans une première extrémité du logement (22').

20. Procédé selon l'une quelconque des revendications 14 à 19, comprenant en outre :
faire passer le premier fil électrique (32) à travers un capuchon de la cage de cheminée (34) ;
le positionnement du clip de cathode (30) sur le capuchon de la cage de cheminée (34) ; et
le soudage du premier fil électrique (32) sur le clip de cathode (30).
